# EUROPEAN PATENT SPECIFICATION

(11) **EP 2 207 486 B1**
(45) Date of publication and mention of the grant of the patent: **21.09.2011**
(21) Application number: 08788995.2
(22) Date of filing: 28.07.2008
(51) Int. Cl.: A61B 17/064, A61B 17/068

(54) **SURGICAL INSTRUMENT FOR FIXING, PREFERABLY IN A MINIMALLY INVASIVE ARTHROSCOPIC MANNER, A CARTILAGINOID TISSUE TO A BONE**
OPERATIONSINSTRUMENT ZUR FIXIERUNG, VORZUGSWEISE AUF MINIMAL INVASIVE ATHROSKOPISCHE WEISE, EINES KNORPELGEWEBES AN EINEM KNOCHEN
INSTRUMENT CHIRURGICAL DESTINÉ À FIXER, DE PRÉFÉRENCE PAR ARTHROSCOPIE MINIMALEMENT INVASIVE, UN TISSU DE TYPE CARTILAGINEUX À UN OS

(30) Priority: 18.09.2007 IT MI20071796
(43) Date of publication of application: 21.07.2010
(73) Proprietor: Pivot Medical, Inc., Sunnyvale, CA 94085 (US)
(72) Inventor: Ganz, Reinhold, 3073 Guemligen (CH)
(74) Representative: Engelhardt & Engelhardt
(86) International application number: PCT/IB2008/002038
(87) International publication number: WO 2009/037535

(56) References cited:
- EP-A- 0 338 779
- GB-A- 2 118 474
- US-A- 5 741 268
- US-A1- 2002 169 465

## Description

### BACKGROUND OF THE INVENTION

The present invention relates to surgical instrument for fixing, preferably in a minimally invasive arthroscopic manner, a cartilaginoid tissue to an underlying bone, with particular reference to an acetabular lip accidentally detached from the osseous edge or rim of a femoral acetabulum. The closest prior art is US 2002/169465 A1, which defines the preamble of claim 1.

As is known, the fixing of an acetabular lip which, for several reasons, has been detached from an underlying bone, is at present carried out by suture anchoring elements, similar to those used in shoulder surgical operations, which frequently require a very invasive so-called "open sky" intervention.

In particular, for applying the above mentioned suture anchoring elements, it is necessary to properly prepare the hip bone, by forming at least a pre-hole and engaging in said pre-hole a dedicated fixation or clamping device.

Then, to the head of the above device one or more suture threads, for fixing the cartilaginoid tissue, for example, to the articular lip, are connected.

For each anchoring element, it is necessary to repeat the just disclosed operations, which greatly extends the surgical operation time.

Moreover, it is very difficult to perform, by the above method, an arthroscopic type of operation and, accordingly, the fixing operation must be carried out by using a substantially conventional type of surgical instrument or tool, with the risk of greatly damaging the underlying bone parts.

### SUMMARY OF THE INVENTION

Accordingly, the main aim of the present invention is to overcome the above mentioned drawbacks of prior fixing methods, by providing a novel fixing surgical instrument, specifically designed to perform a minimally invasive fixing operation, in particular an arthroscopic fixing operation.

Another object of the present invention is to greatly reduce the surgical operation time, while providing a novel surgical fixing or fixation instrument, adapted to perform a much more simple and quick surgical fixing operation.

Another object of the present invention is to provide such a novel surgical instrument providing a safe reliable fixing of the cartilaginoid tissue, and which is construction wise very simple and reliable, and which, moreover, allows to perform a consistently repeatable fixing intervention.

Another object of the present invention is to provide such a minimally invasive quickly operating surgical instrument allowing fixing operations to be performed arthroscopically, thereby greatly reducing possible risks related to the surgical operation, such as an infective, anaesthesia and blood loss risks.

Another object of the present invention is to provide such a surgical instrument capable of fixing the cartilaginoid tissue to the bone without drilling pre-holes or grooves in the bone.

Another object of the present invention is to provide such a surgical instrument allowing to fix the cartilaginoid lip without the need of performing fixing manual operations to clamp, by suture threads, the cartilaginoid tissue to the underlying bone.

Yet another object of the present invention is to provide such a surgical instrument which can be easily used in any operating rooms, requires a minimum maintenance and, moreover, is very competitive from a mere economic standpoint.

According to one aspect of the present invention, the above mentioned objects, as well as yet other objects, which will become more apparent hereinafter, are achieved by a surgical instrument as defined in claim 1.

### BRIEF DESCRIPTION OF THE DRAWINGS

Further characteristics and advantages of the surgical instrument according to the present invention will become more apparent hereinafter from the following detailed disclosure of a preferred, though not exclusive, embodiment thereof, which is illustrated, by way of an indicative, but not limitative example, in the accompanying drawings, where:
Figure 1 is an exploded perspective view, showing the main component parts of the surgical instrument or tool according to the present invention;
Figure 2 is a partial perspective view showing possible geometrical configurations of respective fixing elements which can be applied by the surgical instrument shown in figure 1;
Figure 3 is a detail perspective view showing a possible configuration or embodiment of a cartridge and/or block supporting element, specifically designed for slidably supporting thereon the fixing element or device shown in figure 2;
Figures 4A (from a to c) and 4B (from a' to c') show further partial perspective view of parts of the surgical instrument, according to the present invention, useful for understanding the operation of this instrument;
Figure 5 is a further detail view, as partially cross-sectioned, showing a detail of the surgical instrument according to the present invention, with the fixing device already engaged in its cartridge and cannula and ready for fixing an acetabular lip to an underlying bone (herein not shown);
Figure 6 is yet another perspective view illustrating an upper or top detachment of the acetabular lip;
Figure 7 is yet another perspective view showing the surgical instrument according to the present invention in a ready condition to perform a fixing operation of the acetabular lip; and
Figure 8 is yet another perspective view showing an operating mode of the surgical instrument according to the present invention, after having fixed the acetabular lip to the underlying bone, by applying a single fixing or clamping element or device.

### DESCRIPTION OF THE PREFERRED EMBODIMENTS

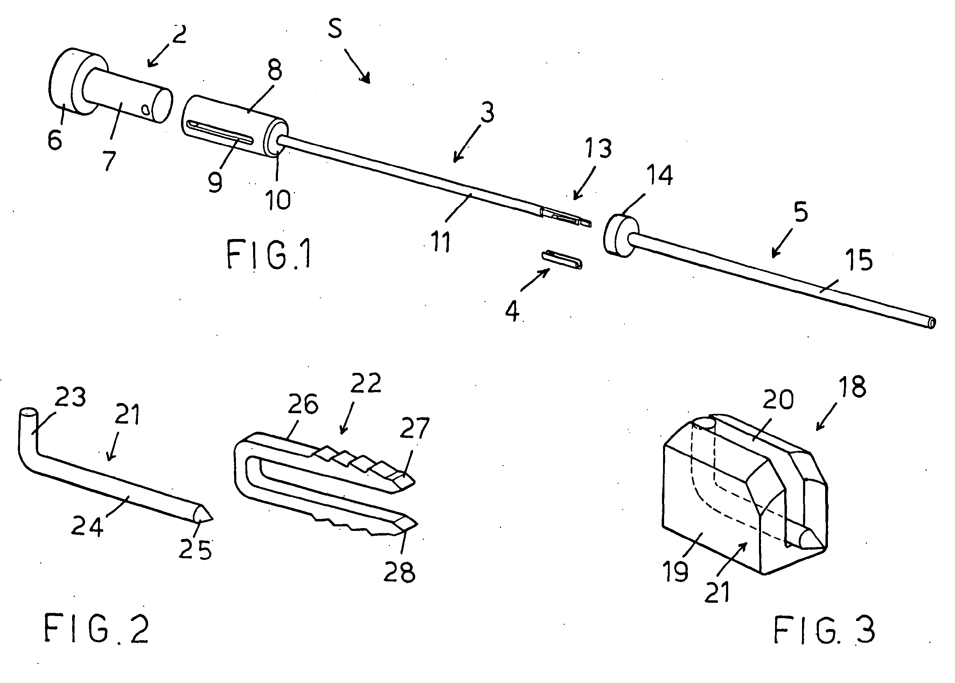

With reference to above mentioned figures, in figure 1 the surgical instrument or tool according to the present invention, has been generally indicated by the reference letter S.

Said instrument comprises a beating mass, generally indicated by the reference number 2, a middle part, generally indicated by the reference number 3, a fixing device bearing carriage, generally indicated by the reference number 4, and a cannula, generally indicated by the reference number 5.

The beating mass 2 of the surgical instrument S comprises, in turn, a substantially solid cylindric beating or impacting head 6, integral with a central stem, also of a substantially solid cylindric configuration, 7, having a diameter less than that of the beating or impacting head 6.

The middle part 3 of the surgical instrument S defines a substantially cylindric hollow proximal end portion 8, having at least a guide slotted longitudinal opening 9, for removably slidingly engaging therein the central stem 7 of the beating mass 2.

Said cylindric hollow portion 8 has an end face 10, operating as a closure bottom for the cylindric portion 8 and therefrom integrally extends a rod-like element, of substantially cylindric configuration 11, having a diameter much smaller than that of the cylindric hollow portion 8, and ending with a substantially flat distal end portion, generally indicated by the reference number 13.

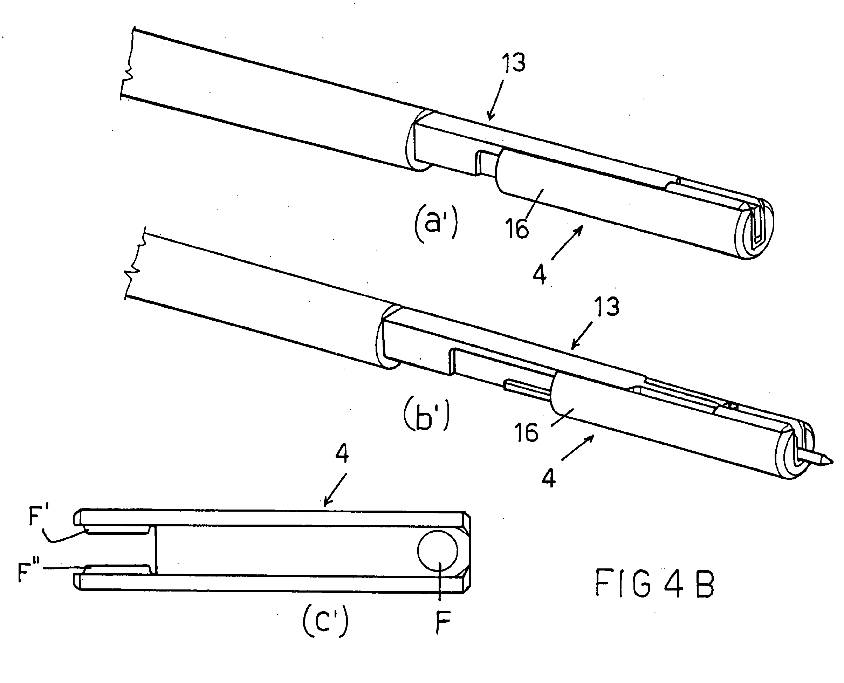

The cannula part 5, in turn, comprises a substantially cylindric hollow head portion 14, designed for abutting, with the rod 11 being engaged in the cannula, against the face 10 of the cylindric hollow portion 8 of the middle part 3, and a cylindric thin cannula portion 15, extending substantially centrally and integrally from the head portion 14 of the cannula 5, and having a diameter substantially less than that of said head portion 14. The carriage element 4, which is shown in a more detailed manner in the perspective view of figure 4 (b) and in the top plan view in figure 4B (c'), comprises, in turn, an elongated cylindric or plug body 16 having a substantially longitudinal central slot 17, for engaging therein a cartridge and/or block element, generally indicated by the reference number 18 in figure 3, having a block body 19 in which is formed a longitudinal slot 20 in which is slidably engaged a fixing device, generally indicated by the reference numbers 21 and 22 in figure 2, respectively.

More specifically, according to a first embodiment thereof, the fixing device 21 comprises a substantially L-shape hook element, having a short arm 23 and a long arm 24.

Advantageously, the long arm 24 ends with a sharpened pointed portion 25.

Said fixing devices 21 and 22 may have different lengths, sizes and cross sections, to perfectly fir specific requirements of the patient, in particular, for properly resisting against any withdrawal and twisting forces applied to the fixing device.

More specifically, the fixing device 21 has a substantially circular cross-sections, whereas the fixing device 22 comprises a clip element 26, of substantially U-shape, and having two respective end spaced tip portions 27 and 28, said clip element 26 further advantageously including surface teeth for improving its anchoring to the bone, after having applied by impacts said clip element 26 to the bone, through the surgical instrument S, as it will become more apparent hereinafter.

According to an important aspect of the present invention, the fixing device, which has substantially a L-hook, or a U-clip shape, comprises moreover, in addition to one or two sharpened tips, a surface textured portion, to prevent it from being accidentally withdrawn from the bone under accidental stresses.

Advantageously, the clamping device 21 or 22 is made of a biocompatible material, adapted to prevent or minimize possible troubles due, for example, to possible clinical analyses, such as NMR analyses, necessary to evaluate a good fixation of the cartilaginoid tissue to the bone and, accordingly, the recovery course of the patient.

Thus, the above mentioned biocompatible material must be of non magnetic nature, and capable to minimize any possible artefacts.

By way of an example, the fixing device can be made of titanium and alloys thereof, biocompatible polymers or other metals or metal alloys, either of a shape memory or of a standard type, provided that they have the above mentioned features.

Thus, the fixing devices 21 or 22, respectively the hook element, or U-shape element, can be engaged in the bone, as it will become more apparent hereinafter, without the need of preliminarily drilling a hole or a recess in said bone, thereby advantageously reducing the operating time and, accordingly, any risks of the surgical intervention (such as infections, anaesthesia or hematic losses).

In figure 3, the fixing device 21 has been shown as slidably supported in said support cartridge 18, which cartridge is advantageously of a disposable type and, in addition to operate for containing the fixing device therein, can be removably slidably engaged in said slot 17 of the body 16 of the carriage 4, to allow the device to be easily handled and engaged by the instrument S.

Actually, as stated, said cartridge and/or block 18 comprises a longitudinal slot 20 restraining therein the fixing device (23 in figure 3) and properly guiding said device as it is impacted into the bone, as it will become more apparent hereinafter.

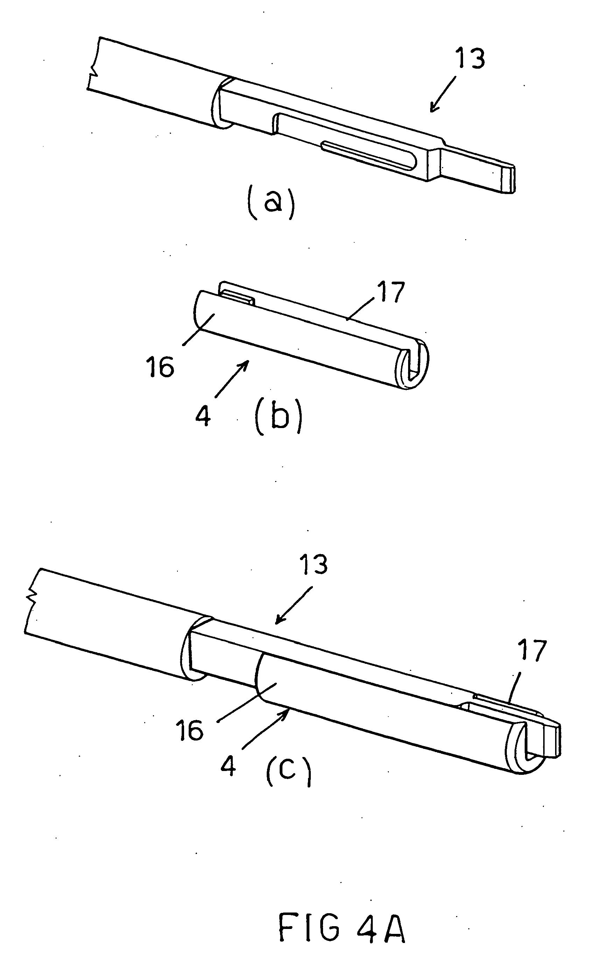

With reference now to figures 4A to 4B, the fixing device supporting carriage 4 can be engaged/assembled in/to the middle part 3, and, in particular, in/to its flat tip portion 13, through the longitudinal slot 17 of the carriage 4.

This coupling to, and a corresponding disengaging from said middle part 3, is performed in a very easy manner by a snap type of operation.

Actually, the carriage 4 can be easily disassembled or detached by removing the fixing device supporting cartridge 18 from its seat (figures 4A, c) (figures 4B, a'), thereby allowing the surgical instrument, and in particular, the middle part 3 thereof, to be easily cleaned and sterilized.

On the contrary, with the cartridge 18 arranged in its seat (figure 4B, b'), said carriage cannot be detached or disassembled, thereby allowing said fixing devices 21 and/or 22, which have a very small size, to be easily handled or driven, thereby preventing them from being accidentally disengaged (figures 4B, c'). Finally, as is shown in figures 4B, c', the carriage 4 also comprises a hole F for allowing the cartridge 18 to be easily removed, as necessary, and suitable machined regions, such as locking ribs F', F", for preventing the cartridge from being ejected in a longitudinal direction.

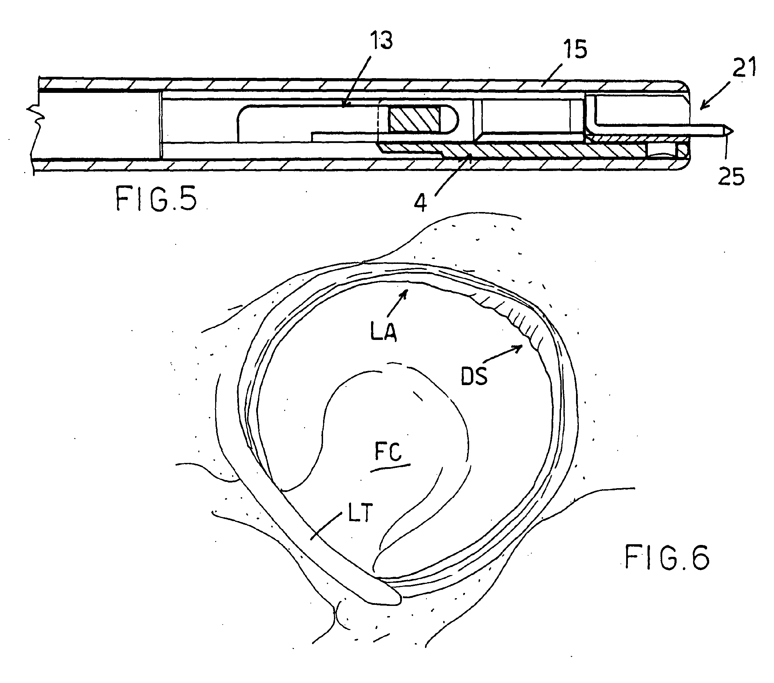

In this connection it should be pointed out that the cartridge 18 for supporting the fixing device 21 and/or 22 will be pre-assembled with the fixing device itself, thereby causing its tip portion 25, or 27 and/or 28, to project from the instrument S in an assembled condition of the latter. Thus, it is possible to easily grip the cartilaginoid tissue and fix this tissue at any desired positions, without using other tools or operators, at it is clearly shown in figure 5.

Thus, the cartridge 18 and inner portion of the surgical instrument S will prevent the device being impacted upon from deviating from a desired operating path. Actually, the instrument S is operatively impacted by causing the beating mass 2, made integral with the inner part 3, to slide in the guide groove and/or elongated slot 9 of the inner part of the instrument S.

Accordingly, as it will become apparent to one skilled in the art, the inventive surgical instrument S allows to implant any desired number of fixing devices 22 and/or 23, without removing said cannula 15, and this by merely withdrawing or removing the inner part 3 and the carriage 4 from the cannula 15, while also removing the empty cartridge 18 (which operation can be easily performed due to the provision of said hole F of the carriage 4), and by engaging a fresh cartridge and related fixing device 21 and/or 22.

Figure 5 is a partial cross-section view illustrating the fixing device 21 supported in its supporting cartridge 18, and engaged in the respective cannula 15.

Figure 6 shows a schematic view of the acetabular lip LA, having an upper or top detached portion DS.

In particular, the cotyloid fossa FC, the articular hyalinic cartilage CIA, and the acetabular transverse ligament LT are herein shown.

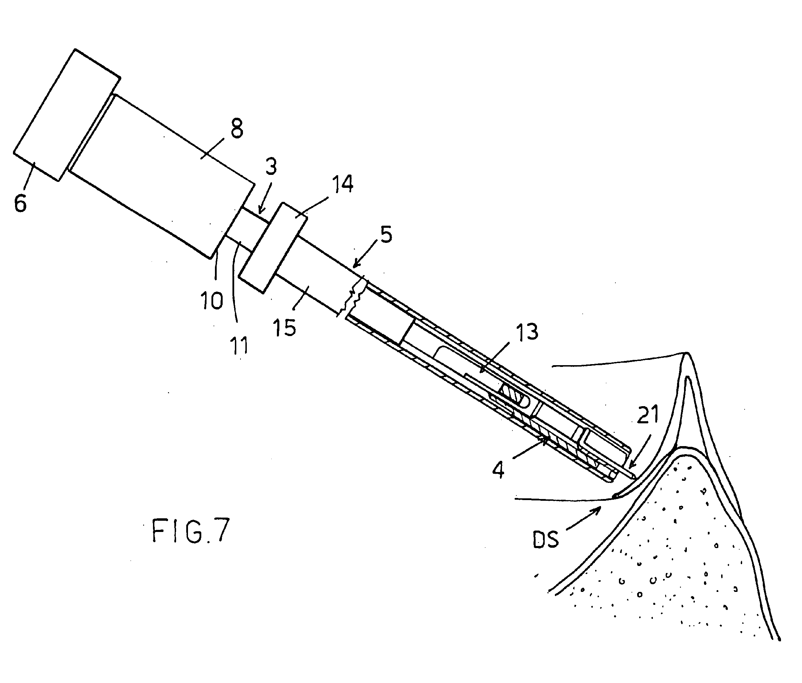

Figure 7 is a schematic view provided for clearly understanding the operation of the inventive surgical instrument S.

In particular, in figure 7, the instrument tp 25 is shown arranged in the cannula, after having engaged the middle part of the instrument S, near the lip L to be fixed.

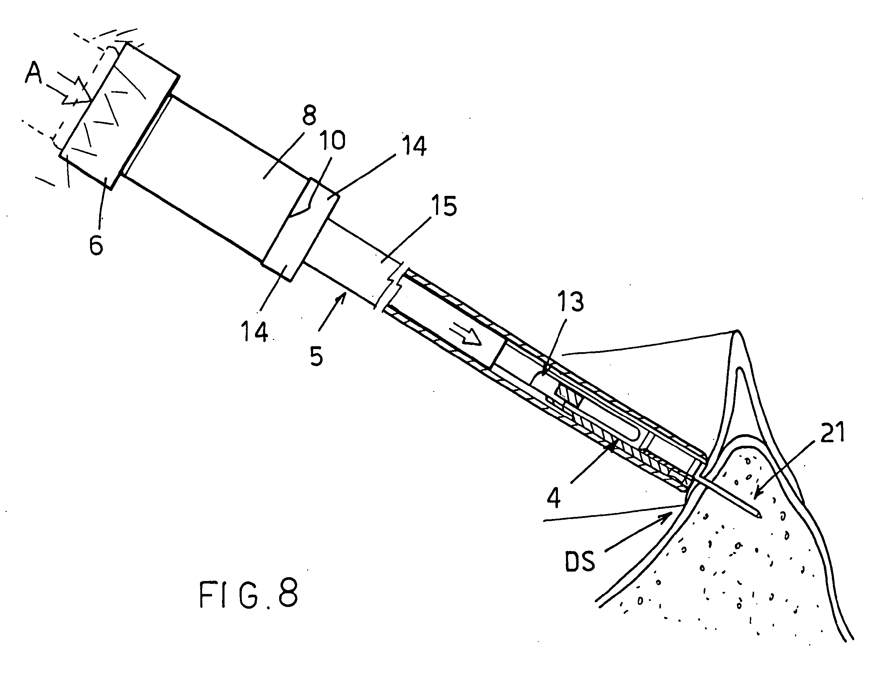

As shown in figure 8, by applying suitable repeated impacts to the head 6 of the beating mass 2 in the direction of the arrow A, for example by a mallet manually driven by the surgeon (not shown), the tip or point portion 5 will be caused to enter both the acetabular lip LA and the underlying bone, thereby completing the fixing operation, by causing the fixing device 21 to be deeply engaged in the bone.

In this connection it should be apparent that this operation can be easily and quickly repeated to provide a perfect fixation by further fixing hook elements 21 to be applied at any desired position, as chosen by the surgeon.

Thus, it is apparent that the inventive surgical instrument S allows to implant any desired number of fixing devices 21 and/or 22, without removing the implanted cannula, but merely withdrawing the inner part 3 and the carriage 4 from the cannula, while removing the empty cartridge 18 (which operation can be easily performed owing to the provision of the carriage hole F), and by introducing a fresh cartridge and related fixing device 21 and/or 22.

From the above disclosure it should be apparent that the invention fully achieves the intended aim and objects.

While the invention has been disclosed with reference to preferred embodiments, it should be apparent that the disclosed embodiments are susceptible to several modifications and variations, all of which will come within the scope of the invention.

In practicing the invention, the used contingent materials and/or sizes and/or shapes can be any, depending on requirements.

## Claims

1. A surgical instrument (S) for fixing in a minimally invasive arthroscopic manner a cartilaginoid tissue acetabular lip (LA) accidentally detached from a bone rim of a femoral acetabulum, said instrument comprising a proximal beating mass (2), having a portion removably engageable with a proximal end of a middle part (3) having a distal end thereof adapted to support a replaceable fixing device (21, 22) which can be removably coupled to said distal end of said middle part (3), and a cannula part (5) adapted to slidably receive therein said middle part (3) with said fixing device (21, 22) removably coupled thereto, **characterized in that** said fixing device (21, 22) is snap removably coupled to said distal end of said middle part (3) by a removably carriage element (4) removably supporting therein said fixing device (21, 22).

2. A surgical instrument according to claim 1, said fixing device (21) comprising an elongated body having at least a pointed and sharpened end portion (25) designed for perforating, as said beating mass (2) is subjected to repeated impacts, said cartilaginoid tissue acetabular lip (LA) and said bone rim, to fix said acetabular lip (LA) to said bone rim, while penetrating said acetabular lip and bone rim, **characterized in that** said elongated body of said fixing device (21) has a substantially L-shape defining L arms having a substantially cylindric cross-section.

3. A surgical instrument according to claim 2, **characterized in that** said fixing device (22) has a substantially U-shape clip configuration, defining two pointed and sharpened portions (26), said U-shape having arms of a substantially rectangular and/or square cross-section.

4. A surgical instrument according to claim 1, **characterized in that** said fixing device (21, 22) is slidably engaged in an engaging slot (17) of a disposable cartridge element (18), said disposable cartridge element (18) being in turn removably slidably engaged in a corresponding longitudinal central slot (17) of said carriage (4), said at least a pointed portion of said fixing device (21, 22) projecting, in an assembled and inoperative condition of said instrument, from a surface of said disposable cartridge element (18).

5. A surgical instrument according to claim 1, said proximal beating mass (2) comprising a substantially cylindric solid impact head (6) integral with a central stem (7), also having a substantially cylindric solid configuration and a diameter much less than a diameter of said impact head (4), **characterized in that** said proximal end of said middle part (3) of said surgical instrument is a substantially cylindric hollow portion (8) having at least a longitudinal guide slot (9) to be removably coupled to said central stem (7) of said proximal beating mass (2), said cylindric hollow portion (8) having a bottom end face therefrom integrally extends a substantially cylindric rod element (11) having a diameter much less than the diameter of said cylindric hollow portion (8) and ending in said distal end of said middle portion (3), said distal end being of essentially flat configuration,

6. A surgical instrument according to claim 1, said cannula part (5) having a substantially cylindric hollow head portion (14) adapted to abut, with said instrument in an assembled condition, against said end surface of said cylindric hollow portion of said middle part (3), and a cylindric thin cannula portion (15) extending substantially centrally integrally from said head portion (14) of said cannula part (5), said cannula part (5) being so designed as to be permanently retained in an implanted condition during a fixation operation, while allowing said fixation operation to be arthroscopically performed in a non-open sky condition, **characterized in that** said carriage element (4) is snap engaged with said flat distal end of said middle part (3), thereby allowing said carriage element (4) to be easily and quickly disassembled as said fixing device (21, 22) supporting cartridge element (18) is removed from said carriage element (4), thereby allowing said surgical instrument to be easily cleaned and sterilized.

7. A surgical instrument according to claim 6, **characterized in that** said carriage element (4) comprises at least a hole (F) to facilitate a removal of said fixing device supporting cartridge element (18), said carriage element (4) being so machined as to present said cartridge element (18) from being ejected in a longitudinal direction.

8. A surgical instrument according to claim 1, **characterized in that** said middle part is so contoured as to be coupled to said carriage element to allow said fixing device to be impacted in a guided manner.

9. A surgical instrument according to claim 1, **characterized in that** said fixing device is implanted by impacts by causing said proximal beating mass (2), made integral with said inner part (3), to slide in a guide slot (9) formed in said inner part (3) of said surgical instrument.

10. A surgical instrument according to claim 1, **characterized in that** said fixing device (21, 22) comprises a macro-textured and/or surface processed surface to prevent said fixing device (21, 22) from being accidentally withdrawn from a respective bone said fixing device is applied to.

11. A surgical instrument according to claim 1, **characterized in that** said fixing device (21, 22) is made of a biocompatible material.

12. A surgical instrument according to claim 11, **characterized in that** said biocompatible material is a non magnetic material selected from titanium and alloys, thereof, biocompatible polymers and/or metals or metal alloys either of a shape memory type or a standard type.

13. A surgical instrument according to claim 1, **characterized in that** said fixing device is so designed as to be implanted upon having gripped said cartilaginoid tissue acetabular lip (LA) through said pointed and sharpened end of said fixing device (21, 22), whereby arranging said cartilaginoid tissue acetabular lip between said bone and the other end portion of said device.

## Patentansprüche

1. Ein chirurgisches Instrument (S) zur Fixierung auf minimal invasive athroskopische Weise eines Knorpelgewebes der Gelenklippe (LA), welches sich zufällig vom Knochenrand des femoralen Acetabulums gelöst hat, wobei besagtes Instrument eine proximale Schlagmasse (2) aufweist, umfasst einen Abschnitt, der auswechselbar in Eingriff mit dem proximalen Endstück eines mittleren Abschnitts (3) steht, mit einem distalen Endstück, das zur Aufnahme eines austauschbaren Fixierelements (21, 22) ausgelegt ist, welches lösbar mit besagtem distalen Endstück des mittleren Abschnitts (3) in Verbindung gebracht werden kann, sowie eine Kanüle (5), welche gleitend besagten mittleren Abschnitt (3) mitsamt des auswechselbar verbundenen Fixierelements (21, 22) aufnehmen kann, **dadurch gekennzeichnet, dass** besagtes Fixierelement (21, 22) leicht auswechselbar in besagtem distalen Endstück des besagten mittleren Abschnitts (3) mithilfe eines ersetzbaren Trägerelements (4), welches die Fixierelemente (21, 22) aufnimmt und deren Austausch erlaubt, gelagert ist.

2. Ein chirurgisches Instrument gemäß Anspruch 1, wobei das Fixierelement (21) einen länglichen Körper umfasst, welcher über mindestens ein spitzes, geschliffenes Endstück (25) verfügt, welches ausgestaltet ist, um besagtes Knorpelgewebe der Gelenklippe (LA) sowie besagten Knochenrand zu perforieren, sobald besagte Schlagmasse (2) wiederholt einer Krafteinwirkung ausgesetzt wird, mit dem Ziel, die besagte Gelenklippe (LA) an besagtem Knochenrand zu fixieren, indes besagte Gelenklippe und Knochenrand durchdrungen werden, **dadurch gekennzeichnet, dass** besagter länglicher Körper des besagten Fixierelements (21) im Wesentlichen L-förmig ausgebildet ist, wobei die bezeichneten L-Arme einen im Wesentlichen zylindrischen Querschnitt besitzen.

3. Ein chirurgisches Instrument gemäß Anspruch 2, **dadurch gekennzeichnet, dass** besagtes Fixierelement (22) im Wesentlichen als U-förmige Klammer ausgestaltet ist und zwei spitze, geschliffene Abschnitte (26) aufweist, wobei die U-Form durch zwei Arme definiert ist, deren Querschnitt im Wesentlichen rechteckig und/oder quadratisch ist.

4. Ein chirurgisches Instrument gemäß Anspruch 1, **dadurch gekennzeichnet, dass** besagtes Fixierelement (21, 22) verschiebbar in die Steck-Aufnahme (17) eines Wegwerf-Steckmoduls (18) eingreift, wobei dieses Wegwerf-Steckmodul (18) wiederum auswechselbar gleitend in eine passende, in der Mitte des Trägerelements (4) befindliche, längliche Aufnahme (17) eingelassen ist, wobei mindestens ein spitzes Endstück des besagten Fixierelements (21, 22) über die Oberfläche des besagten Steckmoduls (18) hinausragt, wenn sich besagtes chirurgisches Instrument im zusammengebauten, inaktiven Zustand befindet.

5. Ein chirurgisches Instrument gemäß Anspruch 1, in welchem besagte Schlagmasse (2), umfassend einen im Wesentlichen zylindrisch ausgebildeten soliden Schlagkopf (6), welcher fest mit einem zentralen Schaft (7), der ebenfalls im Wesentlichen von zylindrischsolider Gestalt ist, allerdings einen wesentlich geringeren Durchschnitt als besagter Schlagkopf (4) aufweist, verbaut ist, **dadurch gekennzeichnet, dass** besagtes proximales Endestück des besagten mittleren Abschnitts (3) des besagten chirurgischen Instruments als ein im Wesentlichen hohlgeformter, zylindrischer Abschnitt (8) ausgebildet ist, welcher über mindestens eine Längsführungsrille (9) verfügt und trennbar mit besagtem zentralen Schaft (7) der besagten proximalen Schlagmasse (2) in Verbindung steht, wobei dieser zylindrische, hohlgeformte Abschnitt (8) eine Stirnfläche an seinem unteren Ende besitzt, von welcher sich ein fest verbautes, im Wesentlichen zylindrisches, stabförmiges Element (11), dessen Durchmesser substanziell geringer ist als der des besagten zylindrischen, hohlgeformten Abschnitts (8), erstreckt, und in besagtes distales Endstück des besagten mittleren Abschnitts (3) ausläuft, wobei besagtes distales Endstück im Wesentlichen eine flache Gestaltgebung zeigt.

6. Ein chirurgisches Instrument gemäß Anspruch 1, in welchem besagtes Kanülstück (5) ein im Wesentlichen hohles Kopfstück (14) hat, welches so ausgebildet ist, dass es, wenn sich das chirurgische Instrument in fertig zusammengebautem Zustand befindet, besagte Stirnfläche des besagten zylindrischen, hohl geformten Abschnitts des besagten mittleren Abschnitts (3) berührt, sowie ein zylindrisches, dünnes Kanülenstück (15), das sich im Wesentlichen in der Mitte des besagten zum Kanülstück (5) gehörenden Kopfstücks (14) befindet und mit diesem fest verbaut ist, wobei besagtes Kanülstück (5) derart gestaltet ist, um für die Dauer eines Fixiervorgangs ununterbrochen im implantierten Zustand zu verbleiben, und sie zugleich gestattet, besagte Fixieroperation arthroskopisch und unter Vermeidung eines Open-Sky-Eingriffs durchzuführen, **dadurch gekennzeichnet, dass** besagtes Trägerelement (4) in besagtes distales, flaches Ende des besagten mittleren Abschnitts (3) einrastet und dabei eine einfache und schnelle Demontage des besagten Trägerelements (4) erlaubt, wenn das besagte das Fixierelement (21, 22) aufnehmende Steckmodul vom Trägerelement (4) entfernt wird, so dass das chirurgische Instrument problemlos gereinigt und sterilisiert werden kann.

7. Ein chirurgisches Instrument gemäß Anspruch 6, **dadurch gekennzeichnet, dass** das besagte Trägerelement (4) mindestens ein Loch (F) umfasst, welches die Entfernung des besagten das Fixierelement aufnehmenden Steckmoduls (18) erleichtert und so verarbeitet ist, dass es ein Hinunterrutschen des besagten Steckmoduls (18) in Längsrichtung verhindert.

8. Ein chirurgisches Instrument gemäß Anspruch 1, **dadurch gekennzeichnet, dass** der besagte mittlere Abschnitt so beschaffen ist, dass er mit dem Trägerelement verkoppelt werden kann, wodurch eine kontrollierte Krafteinwirkung auf besagtes Fixierelement ermöglicht wird.

9. Ein chirurgisches Instrument gemäß Anspruch 1, **dadurch gekennzeichnet, dass** die Implantation des besagten Fixierelements durch Krafteinwirkung erfolgt, wobei besagte Schlagmasse (2), welche fest mit besagtem inneren Abschnitt (3) verbaut ist, veranlasst wird, in eine Führungsrille (9), welche in besagtem inneren Abschnitt (3) ausgebildet ist, hineinzugleiten.

10. Ein chirurgisches Instrument gemäß Anspruch 1, **dadurch gekennzeichnet, dass** besagtes Fixierelement (21, 22) eine makro-strukturierte und/oder bearbeitete Oberfläche aufweist, welche einem versehentlichen Herausziehen des besagten Fixierelements (21, 22) aus einem Knochen, in den dieses Fixierelement eingebracht wird, entgegenwirkt.

11. Ein chirurgisches Instrument gemäß Anspruch 1, **dadurch gekennzeichnet, dass** besagtes Fixierelement (21, 22) aus biokompatiblem Material besteht.

12. Ein chirurgisches Instrument gemäß Anspruch 11, **dadurch gekennzeichnet, dass** besagtes biokompatibles Material nicht magnetische Eigenschaften aufweist und aus Titan und dessen Legierungen, biokompatiblen Polymeren und/oder anderen Metallen oder Metalllegierungen hergestellt ist, wobei sowohl Formgedächtnislegierungen als auch Standardlegierungen einsetzt werden können.

13. Ein chirurgisches Instrument gemäß Anspruch 1, **dadurch gekennzeichnet, dass** besagtes Fixierelement so gestaltet ist, dass es, nachdem es besagtes Knorpelgewebe der Gelenklippe (LA) aufgenommen hat, mithilfe der spitzen, geschliffenen Endstücke des Fixierelementes (21, 22) implantiert werden kann, indem besagtes Knorpelgewebe der Gelenklippe zwischen dem Knochen und dem anderen Endabschnitt der besagten Vorrichtung angeordnet wird.

## Revendications

1. Un instrument chirurgical (S) destiné à fixer par arthroscopie minimalement invasive, une lèvre acétabulaire (LA) de tissu cartilagineux détachée par accident d'un bord osseux ou d'un acétabulum fémoral, ledit instrument comprenant une masse battante (2), ayant une portion amovible insérable avec une extrémité proximale d'une pièce centrale (3) ayant une extrémité distale adaptée pour accueillir un dispositif de fixation remplaçable (21, 22) qui peut être accouplé de manière amovible à ladite extrémité distale de ladite pièce centrale (3), et une canule (5) adaptée pour accueillir par coulissement ladite pièce centrale (3) avec ledit dispositif de fixation (21, 22) qui y est accouplé de manière amovible, **caractérisé en ce que** ledit dispositif de fixation (21, 22) est accouplé de manière amovible à ladite extrémité distale de ladite pièce centrale (3) par un élément de boîtier amovible (4), y accueillant de manière amovible ledit dispositif de fixation (21, 22).

2. Un instrument chirurgical conformément à la revendication 1, ledit dispositif de fixation (21) comprenant un corps allongé ayant au moins une portion finale pointue et acérée (25) conçue pour perforer, tandis que ladite masse battante (2) est soumise à des impacts répétés, ladite lèvre acétabulaire de tissu cartilagineux (LA) et ledit bord d'os, en vue de fixer ladite lèvre acétabulaire (LA) audit bord d'os, pendant la pénétration de ladite lèvre acétabulaire et dudit bord d'os, **caractérisé en ce que** ledit corps allongé dudit dispositif de fixation (21) possède des bras de définition en L avec une forme essentiellement en L ayant une section transversal essentiellement cylindrique.

3. Un instrument chirurgical conformément à la revendication 2, **caractérisé en ce que** ledit dispositif de fixation (22) possède une configuration de clip essentiellement en U, définissant deux portions pointues et acérées (26), ladite forme en U ayant des bras d'une section transversale essentiellement rectangulaire et / ou carrée.

4. Un instrument chirurgical conformément à la revendication 1, **caractérisé en ce que** ledit dispositif de fixation (21, 22) est inséré par coulissement dans une rainure d'insertion (17) d'un élément de cartouche jetable (18), ledit élément de cartouche jetable (18) étant à son tour inséré par coulissement de manière amovible dans une rainure centrale longitudinale (17) correspondante dudit boîtier (4), sachant qu'au moins une portion pointue dudit dispositif de fixation (21, 22) projette, dans un état assemblé et non opérationnel dudit instrument, depuis une surface dudit élément de cartouche jetable (18).

5. Un instrument chirurgical conformément à la revendication 1, ladite masse battante proximale (2) comprenant une tête d'impact solide essentiellement cylindrique (6) intégré dans une tige centrale (7), ayant également une configuration solide essentiellement cylindrique et un diamètre beaucoup plus petit que celui de ladite tête d'impact (4), **caractérisé en ce que** ladite extrémité proximale de ladite pièce centrale (3) dudit instrument chirurgical est une portion creuse essentiellement cylindrique (8) ayant au moins une rainure de guidage longitudinale (9) devant être accouplée de manière amovible à ladite tige centrale (7) de ladite masse battante proximale (2), ladite portion creuse cylindrique (8) ayant une face d'extrémité qui étend à partir de là intégralement un élément de barre essentiellement cylindrique (11) ayant un diamètre bien plus petit que celui de ladite portion creuse cylindrique (8) et se terminant dans ladite extrémité distale de ladite portion centrale (3), ladite extrémité distale étant de configuration essentiellement plate.

6. Un instrument chirurgical conformément à la revendication 1, ladite canule (5) ayant une portion de tête creuse essentiellement cylindrique (14) conçue pour abouter, avec ledit instrument en état assemblé, contre ladite surface terminale de ladite portion creuse cylindrique de ladite pièce centrale (3), et une portion de canule fine cylindrique (15) intégrée s'étendant essentiellement centralement depuis ladite portion de tête (14) de ladite canule (5), ladite canule (5) étant conçue de manière à être retenue en permanence dans un état implanté durant une opération de fixation, tout en permettant à ladite opération de fixation d'être réalisée de manière arthroscopique dans un état autre qu'à ciel ouvert, **caractérisé en ce que** ledit élément de boîtier (4) est inséré par encliquetage avec ladite extrémité distale plate de ladite pièce centrale (3), permettant ainsi audit élément de boîtier (4) d'être démonté facilement et rapidement alors que ledit dispositif de fixation (21, 22) supportant l'élément de cartouche (18) est retiré dudit élément de boîtier (4), permettant ainsi audit instrument chirurgical d'être nettoyé et stérilisé facilement.

7. Un instrument chirurgical conformément à la revendication 6, **caractérisé en ce que** ledit élément de boîtier (4) comprend au moins un trou (F) pour faciliter un retrait dudit élément de cartouche (18) supportant le dispositif de fixation, ledit élément de boîtier (4) étant usiné de manière à éviter que ledit élément de cartouche (18) soit éjecté dans le sens longitudinal.

8. Un instrument chirurgical conformément à la revendication 1, **caractérisé en ce que** ladite pièce centrale possède des contours lui permettant d'être accouplée audit élément de boîtier pour permettre audit dispositif de fixation d'appliquer un impact de manière guidée.

9. Un instrument chirurgical conformément à la revendication 6, **caractérisé en ce que** ledit dispositif de fixation est implanté par des impacts en causant que ladite masse battante proximale (2) intégrée avec la pièce intérieure (3) coulisse dans une rainure de guidage (9) formée dans ladite pièce intérieure (3) dudit instrument chirurgical.

10. Un instrument chirurgical conformément à la revendication 1, **caractérisé en ce que** ledit dispositif de fixation (21, 22) comprend une surface macro-texturée et / ou une surface traitée pour éviter que ledit dispositif de fixation (21, 22) soit retiré accidentellement de l'os correspondant auquel ledit dispositif est appliqué.

11. Un instrument chirurgical conformément à la revendication 1, **caractérisé en ce que** ledit dispositif de fixation (21, 22) est constitué d'un matériau biocompatible.

12. Un instrument chirurgical conformément à la revendication 11, **caractérisé en ce que** ledit dispositif de fixation (21, 22) ledit matériau biocompatible est un matériau non magnétique sélectionné à partir de titane et d'alliages de titane, de polymères biocompatibles et / ou de métaux ou d'alliages de métaux soit à mémoire de forme ou de type standard.

13. Un instrument chirurgical conformément à la revendication 1, **caractérisé en ce que** ledit dispositif de fixation est conçu pour être implanté après avoir agrippé ladite lèvre acétabulaire (LA) du tissu cartilagineux à l'aide de l'extrémité pointue et acérée dudit dispositif de fixation (21, 22), plaçant ainsi ladite lèvre acétabulaire du tissu cartilagineux entre ledit os et l'autre portion finale dudit dispositif.
